# EUROPEAN PATENT APPLICATION

(11) **EP 2 942 063 A1**
(43) Date of publication of application: **11.11.2015**
(21) Application number: 15166581.7
(22) Date of filing: 06.05.2015
(51) Int. Cl.: A61K 47/32, A61K 31/722, A61P 11/12

(54) **Composition for fluidizing mucus comprising chitosan and copolymers**

(30) Priority: 06.05.2014 IT MI20140825
(71) Applicant: S.I.I.T. S.r.L. - Unipersonale, 20090 Trezzano Sul Naviglio (MI) (IT)
(72) Inventor: Costa, Andrea, 20090 Trezzano Sul Naviglio (MI) (IT)
(74) Representative: Hoffmann Eitle

(57) **Abstract**

The present invention relates to a liquid fluidifying composition for use as nasal spray, for oral or topical use containing a plant chitosan. Furthermore, the present invention relates to a process for preparing said liquid fluidifying composition. Finally, the present invention relates to the use of said liquid fluidifying composition, preferably as nasal spray, for oral or topical use in the treatment of cold weather ailments, catarrh, common cold, flu, nasal infections, rhinitis, allergic rhinitis and sinusitis.

## Description

The present invention relates to a liquid composition for fluidifying mucus for use as nasal spray, for oral or topical use, containing a chitosan, preferably a plant chitosan. Furthermore, the present invention relates to a process for preparing said liquid fluidifying composition. Finally, the present invention relates to the use of said liquid composition, preferably as nasal spray, for oral or topical use, for the treatment of cold weather ailments, catarrh, common cold, flu, nasal infections, rhinitis, allergic rhinitis and sinusitis.

Catarrhal conditions in general, and specifically rhinitis, are known to be among the most common respiratory disorders, which very often also affect adolescent and pediatric subjects. The most common symptoms are nasal congestion and irritation of the respiratory tract, particularly nose and throat.

Suitable formulations for providing a symptomatic benefit to subjects affected, for example, by rhinitis or cold weather ailments and diseases are commercially available. These formulations are represented by topical decongestants. However, in the case of pediatric or adolescent subjects, said formulations have some drawbacks and side effects, which limit the use thereof.

Formulations with mucolytic action, which directly act fluidifying the mucus, are also commercially available. Nevertheless, these formulations also have drawbacks, which limit the use thereof.

Therefore, there is still a strong need by medical professionals to have novel formulations/compositions and new pharmaceutical forms for administration, which would allow to obtain a fast removal of an excess nasal secretion, due to the overproduction of mucus and mucin, occurring during cold weather ailments, catarrh, common cold, flu, nasal infections, rhinitis, allergic rhinitis and sinusitis.

Following to a long and intensive research and experimental activity, the Applicant developed a new liquid composition for fluidifying mucus for use as nasal spray, for oral or topical use, able to suitably meet the limits and drawbacks of the commercially available formulations, in particular those recommended for pediatric or adolescent subjects. The composition of the present invention is effective, well tolerated in children and stable.

It is an object of the present invention a liquid fluidifying composition for use as nasal spray, for oral or topical use, having the characteristics as claimed in the appended independent claim.

It is an object of the present invention a liquid fluidifying composition for use as nasal spray, for oral or topical use, for use in the treatment of cold weather ailments, catarrh, common cold, flu, nasal infections, rhinitis, allergic rhinitis and sinusitis, having the characteristics as claimed in the appended independent claim.

It is an object of the present invention a process for preparing said liquid fluidifying composition having the characteristics as claimed in the appended independent claim.

It is an object of the present invention the use of said nasal fluidifying composition as medical device or pharmaceutical composition for the treatment of cold weather ailments and diseases, common cold, flu, nasal infections, rhinitis, allergic rhinitis and sinusitis, having the characteristics as claimed in the appended independent claim.

Other preferred embodiments are described in the following detailed description.

Said liquid fluidifying composition can be in a pharmaceutical form for oral or topical use, such as a nasal spray, a solution, a drop solution or a syrup.

The Applicant found the use of a chitosan to be useful and convenient. Chitosan can be of animal or plant origin, preferably it is a plant chitosan. The selected chitosan can be, for example, of food grade and can be, for example, Kionutrime-CsG™ (plant biopolymer consisting of N-acetyl-D-glucosamine and D-glucosamine) (Kitozyme SA) CAS [9012-76-4]: ethanol-insoluble, but soluble in slightly acidic solutions; chitosan content greater than or equal to 80% by weight, relative to the dry weight, preferably greater than or equal to 85% by weight, relative to the dry weight (KZ PT-CQ-124 method), beta-glucan content less than or equal to 10% by weight, relative to the dry weight, preferably less than or equal to 15% by weight, relative to the dry weight (KY PT-cQ-111 method); loss on drying less than or equal to 10% by weight, relative to the wet weight (KZ PT-CQ-100 method); degree of acetylation (mol%, relative to the wet weight) from 0 to 30% (KZ PT-CQ-101 method); 1% viscosity, in 1% HAc, (mPa·s) comprised from 1 to 15 (KZ PT-CQ-102 method); granulometry (% by weight) greater than 95%, 250 µm (KZ PT-CQ-117), preferably granulometry D0.9 (µm) less than or equal to 250 (particle size analysis by laser diffraction, Mastersizer 2000, ISO 13320; tapped density comprised from 0.7 to 1 g/cm³ (EP 2.9.34).

Chitosan, preferably of plant origin, being used in the present invention has an average molecular weight comprised from 10 to 80 KDa, preferably from 15 to 50 KDa, even more preferably from 20 to 40 KDa; advantageously, it has an average molecular weight comprised from 10 KDa to 30 KDa, preferably 15 KDa or 20 KDa.

Chitosan being used may have a degree of acetylation of less than 50%, preferably comprised from 0 to 30%. Advantageously, the plant chitosan herein used has an degree of acetylation comprised from 0 to 30 by %mol, relative to the wet weight (side groups hindered by the presence of the acetyl group and, thus, no longer free alkaline groups); in this case, plant chitosan herein used has a degree of deacetylation greater than or equal to 70 by mol%, relative to the wet weight, such as in the case of the plant chitosan Kionutrime-CsG® (Kitozyme SA).

Said chitosan, preferably of plant origin, is contacted and mixed with a selected polymeric emulsifier selected from the group comprising, or alternatively, consisting of: cross-linked copolymers with INCI name Acrylates/C-10-30 Alkyl Acrylate Crosspolymer. Known cross-linked copolymers (group (i)) with INCI name Acrylates/C-10-30 Alkyl Acrylate Crosspolymer are: Carbopol^{®} 1342 Polymer, Carbopol^{®} 1382 Polymer, Carbopol^{®} ETD 2020 Polymer, Carbopol^{®} SC-200 Polymer, Carbopol^{®} Ultrez 20 Polymer, Carbopol^{®} Ultrez 21 Polymer, Pemulen™ TR-2 Polymeric Emulsifier and Pemulen™ TR-1 Polymeric Emulsifier, (INCI means -International Nomenclature of Cosmetic Ingredients). The Patent Applications US 5,322,689 A, US 5,591,236 A, US 4,758,641 A and US 5,004,557 A are herein incorporated by reference, as regards the polymeric emulsifier Pemulen, known as poly(acrylic acid) polymers whit high molecular weight.

Advantageously, the polymeric emulsifier being used is a cross-linked copolymer selected from Pemulen™ TR-2 Polymeric Emulsifier and/or Pemulen™ TR-1 Polymeric Emulsifier. The article available on www.lubrizol.com concerning the Technical Data Sheet TDS-114 entitled *"Introducing Pemulen Polymeric Emulsifiers"* is herein incorporated by reference.

Carbopol®-type copolymers are flocculated powders of particles with an average diameter of 0.2 microns. Flocculated powders with an average diameter from 2 to 7 microns are determined by means of Coulter Counter. Once produced, these agglomerates cannot be broken or size-reduced down to the primary particles. Each particle can be schematized as a network structure of polymeric chains interconnected by cross-linking. Carbopol® 907 is a linear and water-soluble copolymer (Technical data sheet TDS-222). The other Carbopol® copolymers, Pemulen™ and Noveon®, are cross-linked and, in an environment with a pH from 4 to 6, swell in water up to 1000 times their original volume and 10 times their original diameter to form a gel. Cross-linked polymers do not dissolve in water, due to their three-dimensional molecular structure (TDS-222). Polymers similar to Carbopol polymers are also known as Carbomer, a polymer family with hydrophilic nature, which allows them to absorb water, hydrate and swell. Carbopol polymers have an average weight equal to 76 carboxylic groups.

USP-NF, *European Pharmacopoeia, British Pharmacopoeia, United States Adopted Names Council* (USAN) and *International Nomenclature for Cosmetic Ingredients* (INCI) adopted the generic name "Carbomer" for several kinds of polymers Carbopol homopolymers, which share chemical and physical characteristics. The Japanese Pharmacopoeia states adopted for Carbopol homopolymers the name "carboxyvinyl polymer" and "carboxy polymethylene". The Italian Pharmacopoeia identifies Carbopol 934P as "carboxy polymethylene" and the Deutschen Artzneibuch refers to Carbopol 980NF as "polyacrylic acid." Carbopol copolymers such as Carbopol 1342 NF and 1382 have been named "carbomer" by USP-NF, but they are considered as "Acrylates/C10-C30 Alkyl Acrylates Cross polymer" by INCI.

Chitosan, such as the plant chitosan, is contacted or added to an emulsifying compound selected from the group comprising or, alternatively, consisting of cross-linked copolymers of acrylic acid and C10-C30 alkyl acrylates (Acrylates/C10-C30 Alkyl Acrylates Cross polymer), selected from those described above. Said cross-linked copolymers are selected from the group comprising or, alternatively, consisting of Pemulen™ TR-2 Polymeric Emulsifier and Pemulen™ TR-1 Polymeric Emulsifier or mixtures thereof (Lubrizol Advanced Materials, Inc.) (TDS-222).

In an embodiment said Pemulen™ polymer is the polymer Pemulen™ 1621 Polymer: high average molecular weight; viscosity: emulsion viscosity, 0.2% emulsion in 20% mineral oil, Brookfield RVT, (mPa·s)(20 rpm at 25°C) from min. 2 to max. 12 (TP-SA-015); Oil loading up to 30%; Moisture Content (Loss on Drying) max. 3% by weight (TP-SA-004); concentration of use from 0.2 to 0.5%.

In another embodiment said Pemulen™ polymer is the polymer Pemulen™ 1622 Polymer: high average molecular weight; low viscosity: emulsion viscosity, 0.2% emulsion in 20% mineral oil, Brookfield RVT, (mPa·s)(20 rpm at 25°C) from min. 1.5 to max. 4 (TP-SA-015); Oil loading up to 70%; Moisture Content (Loss on Drying) max. 3% by weight (TP-SA-004); concentration of use from 0.15 to 0.3%.

In an embodiment said Pemulen™ copolymer is, advantageously, the copolymer Pemulen™ TR-2 Polymeric Emulsifier (INCI Name: Acrylates/C10-30 Alkyl Acrylate Crosspolymer) (TDS-114), it contains the highest level of hydrophobic groups and can emulsify the highest level of oil (up to 50% by weight) with a pH range of 4-5; emulsion viscosity (mPa·s) (Brookfield RVT or RVF at 20 rpm, 25°C, #4 spindle) 0.2% solution from min. 1.700 to max. 4.500 (Test Procedure-SA-015); Moisture Content max. 2 (TP-SA-004); Mucilage Viscosity (mPa·s) Brookfield RVT or RVF at 20 rpm, 25°C, #4 spindle, 0.2% solution from min. 1.050 to max. 3.750 (TP-430-I); carboxylic acid content comprised from 52 to 62% (USP/NF); Loss on drying 2% max. (USP/NF); residues of ethyl acetate solvent 0.30% max, cyclohexane 0.30% max, benzene 0.50 ppm max. (Lubrizol SA-009); free acrylic acid 2.500 ppm max. (USP/NF). The copolymer Pemulen™ TR-2 Polymeric Emulsifier is described in the United States Pharmacopeia/National Formulary (USP/NF) monograph for Carbomer Copolymer Type A.

In another embodiment said Pemulen™ copolymer is, advantageously, the copolymer Pemulen™ TR-1 Polymeric Emulsifier (INCI Name: Acrylates/C10-30 Alkyl Acrylate Crosspolymer) and has the following characteristics: emulsion viscosity, cP, at 25°C (Brookfield RVT at 20 rpm, by neutralizing the pH between 7.3 and 7.8) solution 0.2% by weight, spindle#5 comprised from 6.500 to 15.500 (Lubrizol SA-015) and 1% by weight of mucilage, spindle#6 comprised from 10.000 to 26.500 (Lubrizol 430-I); Loss on drying 2% max. (USP/NF); residues of ethyl acetate solvent 0.30% max., cyclohexane 0.30% max. and benzene 0.50 ppm max. (Lubrizol SA-009 and SA064); free acrylic acid 2.500 ppm max. (Lubrizol SA-005). The copolymer Pemulen™ TR-1 Polymeric Emulsifier is described in the United States Pharmacopeia/National Formulary (USP/NF) monograph for Carbomer Copolymer Type B.

The concentration of use is from 0.01% to 0.5% by weight, preferably from 0.05% to 0.4%; even more preferably from 0.1% to 0.3%, yielding low-viscosity emulsions.

In another embodiment, chitosan, such as the plant chitosan, is contacted or added to an emulsifier selected from the group comprising or, alternatively, consisting of cross-linked acrylic acid homopolymers. Said homopolymers (group (ii)) are selected from the group comprising or, alternatively, consisting of homopolymers with INCI name Carbomer. Known homopolymers with INCI name Carbomer are: Carbopol^{®} 2984 Polymer, Carbopol^{®} 5984 Polymer, Carbopol^{®} 934 Polymer, Carbopol^{®} 940 Polymer, Carbopol^{®} 941 Polymer, Carbopol^{®} 980 Polymer, Carbopol^{®} 981 Polymer, Carbopol^{®} ETD 2050 Polymer, Carbopol^{®} Silk 100 Polymer, Carbopol^{®} Ultrez 10 Polymer, Carbopol^{®} Ultrez 30 Polymer, (Lubrizol Advanced Materials, Inc.). The Patent Applications CA 2799942 A and WO 2012087325 A1 are herein incorporated by reference, as regards Carbomer homopolymers.

In an embodiment said Carbopol^{®} polymer is the polymer Carbopol^{®} Ultrez 30 Polymer, emulsion viscosity (mPa·s) (Brookfield RVT or RVF at 20 rpm, 25°C, spindle #7) 0.5% solution from min. 45 to max. 65 (TP-430-I), dispersing time 15 minutes; Loss on Drying/Moisture Content max. 2 (TP-SA-004).

The concentration of use is from 0.01% to 1.5% by weight, preferably from 0.05% to 1%; even more preferably from 0.1% to 0.5%, yielding low- and medium-viscosity emulsions (TDS-927, TDS-222).

It is an object of the present invention a process for preparing said liquid fluidifying composition, which consists of the following steps.

In un first vessel a gel-like solution, comprising water in an amount by weight comprised from at least 90% to 99.99% by weight, preferably from 94 to 96% by weight, for example 95% or, alternatively, water and glycerin (in an amount by weight comprised from 60 to 80% of water, preferably 70% water and 30% glycerin) in an amount comprised from at least 95% to 99.99% and an emulsifying compound such as (i) one or more cross-linked copolymers of acrylic acid and C10-C30 alkyl acrylates (INCI Acrylates/C10-C30 Alkyl Acrylates Cross polymer) as identified above and/or (ii) one or more cross-linked acrylic acid homopolymers (INCI Carbomer), as identified above, is prepared. Advantageously, Pemulen™ TR-2 Polymeric Emulsifier and/or Pemulen™ TR-1 Polymeric Emulsifier is used. It has to be noted that cross-linked copolymers of group (i) are not interchangeable or replaceable with homopolymers of group (ii), the latter being preferred and thus absent.

The aqueous or, alternatively hydroglyceric solution/gel, obtained with Pemulen™ TR-2 Polymeric Emulsifier and/or Pemulen™ TR-1 Polymeric Emulsifier has a pH comprised from 3 to 5, preferably comprised from 3.5 to 4.5, and is stirred over a period of time comprised from 1 to 3 hours, preferably 2 hours, at a temperature comprised from 40 to 60°C, preferably 50°C. Said solution containing water in an amount by weight comprised from 90% to 99.99%, preferably from 90% to 97%, for example 99.95%, or a hydroglyceric solution, and said emulsifying compound in an amount by weight comprised from 0.1% to 0.01%, preferably from 0.08% to 0.02%.

In a second vessel a dispersion comprising a chitosan, preferably a plant chitosan, and water is prepared. The resulting dispersion has an alkaline pH, for example comprised from 8 to 10, preferably around a pH value of about 9, depending on the degree of deacetylation of the plant chitosan being used. For example a chitosan with a degree of deacetylation of 70% has a pH value of about 9 in water, whereas a chitosan with a degree of deacetylation of 90% has a pH value of about 9.5-10 in water. The above dispersion is kept under continuous stirring at a temperature comprised from 30 to 40°C.

Said chitosan and water dispersion contains water in an amount by weight comprised from 95% to 99%, preferably from 97% to 99.95%, and said chitosan in an amount by weight comprised from 0.25% to 0.05%, preferably from 0.08% to 0.02%.

The dispersion of the second vessel is kept under stirring and added with a strong acid such as, for example, hydrochloric acid, for example 1 M, since it is fundamental to have a dispersion pH comprised from 6.5 to 8, preferably from 7 to 8, prior to adding said dispersion to said first vessel content. By operating in this way, a deposit or precipitate formation is avoided. In order to best refine the pH value of the dispersion in a range comprised from 6.5 to 8, preferably from 7 to 8, a strong base such as, for example, NaOH, for example 1 M or a 30% solution can be also used, following to the strong acid.

Preferably the weight ratio between said emulsifying compound (i) or (ii) and chitosan is from 1:5 to 1:3.

Chitosan (linear polymers of beta-(1,4)-D-glucosamine) can be an animal or plant chitosan. Advantageously, it is a plant chitosan. Chitosan, preferably of plant origin, being used in the present invention has an average molecular weight comprised from 10 to 80 KDa, preferably from 15 to 50 KDa, even more preferably from 20 to 40 KDa; advantageously, it has an average molecular weight comprised from 10 KDa to 30 KDa, preferably 15 KDa or 20 KDa. The chitosan herein used may have a degree of acetylation of less than 50%, preferably comprised from 0 to 30%. Advantageously, the plant chitosan herein used has a degree of acetylation comprised from 0 to 30 by mol%, relative to the wet weight; in this case the plant chitosan herein used has a degree of deacetylation greater than or equal to 70 by mol%, relative to the wet weight, such as in the case of the plant chitosan Kionutrime-CsG® (Kitozyme SA).

Plant chitosans being effectively applied in the present invention are selected from the group of chitosans with the following characteristics:
- ethanol-insolubility,
- solubility in slightly acidic solutions, pH from 6 to 7,
- chitosan content greater than or equal to 80% by weight,
- degree of acetylation (mol%) comprised from 0 to 50%, preferably from 0 to 30%,
- average molecular weight comprised from 10 to 80 KDa, preferably from 15 to 50 KDa, even more preferably from 20 to 40 KDa; advantageously, it has an average molecular weight comprised from 10 KDa to 30 KDa, preferably 15 KDa or 20 KDa,
- preferably, a granulometry (% by weight) greater than 95%, 250 µm.

In a preferred embodiment, said plant chitosan is Kionutrime-Cs™ (plant biopolymer consisting of N-acetyl-D-glucosamine and D-glucosamine) (Kitozyme SA) CAS [9012-76-4].

Chitosan is a biodegradable and hydrophilic polymer with an average molecular weight of about 650 kDa and a pKa of about 6.5. Chitosan is soluble at acidic and slightly acidic pH values (insoluble at pH 7) and, in the presence of water, it forms a gel with a marked water-retention ability. When chitosan goes into solution due to the presence of positive charges of amino groups, it becomes mucoadherent since interacts with the negative charges of sialic acid residues of the mucosa. The plant chitosan at pH comprised from 6 to 7 (with a slight, partial positive charge) behaves as a "non-destructive" mucolytic agent with mucin (having a slight, partial negative charge). The interaction degree with mucin depends on many factors such as, for example, the amount of sialic acid of mucin, the molecular weight and degree of deacetylation of chitosan, and the spatial conformation of chitosan being used. Interactions are greater at acidic pH because of a greater charge density. However, the problem arises from the fact that the use of a composition comprising chitosan at acidic pH is not possible, since said acidity would lead to an unusable product, because it would cause burning sensation and damages when contacted with respiratory tract tissues (nose and throat).

Next, said aqueous or, alternatively, hydroglyceric solution/gel being present in said first vessel is added to said dispersion of said second vessel, whose pH value was brought to a value comprised from 6.5 to 8, preferably from 7 to 8, to form a stable chitosan suspension with a pH value comprised from 6 to 7, preferably about 6.5, which dissolves due to the presence of the emulsifier, Pemulen™ TR-2 Polymeric Emulsifier and/or Pemulen™ TR-1 Polymeric Emulsifier, and the combined action with a properly adjusted pH. The stable suspension being obtained has a viscosity value comprised from 50 to 1500 cPs, preferably from 200 to 800 cPs. In the case that the pH of the stable suspension is not comprised from 6 to 7, preferably about 6.5, it can be optionally adjusted by adding a pH adjuster. After that, the resulting stable suspension can be optionally diluted up to 4-fold and/or a preservative, flavors and sweeteners can be added.

The osmotic concentration value (measured in milliosmoles) in the above stable suspension is of less than 50 mOsmoles, preferably of less than 40 mOsmoles and greater than 5 mOsmoles.

It is important that the final osmotic concentration value of the stable suspension is comprised from 50 mOsmoles (hypotonic) to 300 mOsmoles (hypertonic). For this aim, an inorganic salt (electrolyte), such as NaCl, is added to said stable suspension.

The final osmotic concentration value, comprised from 50 mOsmoles (hypotonic) to 300 mOsmoles (hypertonic), of the stable suspension, derives from the addition of the strong inorganic acid, such as HCl and the strong inorganic base, such as NaOH, if required, and then, the addition of an inorganic salt (electrolyte), such as NaCl.

In a preferred embodiment, the salt, such as NaCl, being added is in an amount comprised from 0.1% to 3% by weight, preferably in an amount comprised from 0.5% to 2.2% by weight, relative to the final weight of the final suspension.

It is an object of the present invention the use, as medical device or pharmaceutical product, of the liquid fluidifying composition of the present invention for all those cases in which it is desirable to obtain a fast removal of an excessive secretion, formed at respiratory or nasal tracts, due to the overproduction of mucus, occurring during cold weather ailments, catarrh, common cold, nasal infections, rhinitis, allergic rhinitis and sinusitis.

Mucus is a colloidal, viscous secretion, rich in glycoproteins (glycoconjugates), water and salts covering the body's mucous membranes. The rheological properties of mucus depend on many factors such as pH (at lower pH mucus is more viscous), the presence of salts and interactions with endogenous substances. Mucus exerts, among others, both a lubricant and biological barrier functions against infectious agents. Mucus is also produced as thin layer in nose and throat. When infectious, allergenic or irritant agents trigger an inflammatory reaction, organisms react by enhancing the mucus and mucin secretion, causing the congestion of respiratory tract, nose and throat, resulting in difficulty breathing. Mucin comprises 17 members of large, negatively charged and highly glycosylated proteins, with a molecular weight comprised from 1000 to 40000 kDa. The characteristic of mucins in an aqueous medium is represented by their ability to aggregate and form a gel with lubricant and pathogen-barrier functions.

At physiological pH, due to the presence of sialic acid and sulfate residues of mucins, the mucus adhering to the mucosa carries a partial negative charge. Under these conditions the mucus, when contacted, for example, with a partially positively charged macromolecule, can give rise to an interaction known as mucoadhesion. Among mucoactive agents, mucolytics are known, which act by breaking down the chemical bonds of mucus macromolecules, thus reducing the mucus viscosity. But "non-destructive" mucolytics are also known, which act by dissociating or breaking down the mucin network by means of a mechanism known as "charge shielding".

The liquid fluidifying composition (stable dispersion) of the present invention for use as nasal spray, for oral or topical use, being prepared according to the above process, can be added with known preservatives such as polylysine or sodium paraben. However, it is essential that an increase of the osmotic concentration of the composition does not occur; accordingly, salts and all the additives or formulation technological compounds which can dissociate and lead to an increase of free charges in the liquid composition of the present invention have to be avoided.

The composition of the present invention comprising a mixture which comprises or, alternatively, consists of a chitosan, advantageously, a plant chitosan as described above, and (i) one or more cross-linked copolymers of acrylic acid and C10-C30 alkyl acrylates (Acrylates/C10-C30 Alkyl Acrylates Cross polymer) -as identified above, or (ii) one or more cross-linked acrylic acid homopolymers -as identified above, is applied, for example, in the nose by a spray system nebulizing said composition which spreads and distributes throughout the nasal walls. In the presence of mucus and mucin, the composition is contacted with the mucus and mucin and an interaction among them occurs. After a variable contacting time, depending on mucus and mucin amounts, the composition of the present invention dissolves mucin, thus exerting a fluidifying action. Basically, mucin displaces the emulsifier of the composition of the present invention, which interacted, by acid-base attraction, with chitosan groups, and binds chitosan with a greater affinity than the emulsifier. Mucins compete with acrylic polymer and bind chitosan in a more stable manner than the acrylic polymer. Chitosan causes, when bound to mucin, the precipitation thereof, and thus it is not longer available as mucus viscosizing agent.

The Applicant carried out viscosimetry tests for some mixtures comprising the composition of the present invention (0.16% content (130 microliters) of a plant chitosan with a degree of acetylation equal to or less than 50% and an average molecular weight of 25-30 KDa, and 5 variable concentrations of bovine submaxillary mucin type I-S (0.5%, 1%, 2%, 4%, 6%). The rheological interactions at neutral pH and T=37°C with mucin were measured, in order to determine the trend of the zero viscosity point (Method described by Hassan and Gallo (1990), Ferrari et al. (1997) and Rossi et al. (2001), deltaη = ηmix-(ηpol + ηmuc), wherein ηmix is the apparent viscosity of the mixture polymeric emulsifier-mucin (Pa·s), ηpol is the apparent viscosity of a polymeric solution having the same concentration as the mixture (Pa·s), ηmuc is the apparent viscosity of the mucin dispersion having the same concentration as the mixture (Pa·s). The shear rate was selected so that to maximize the deltan parameter. A [composition:mucin] ratio close to that of physiological conditions which implies the admixture of 130 microliters of composition (corresponding to an adult dose) with 1.17 ml of an aqueous mucin solution at 2% weight/weight was used. Viscosimetry measurements were carried out by a rotational rheometer on: (i) aqueous mucin solutions, (ii) composition mixed with an aqueous mucin solution and (iii) water diluted formulations under the same conditions for the mixture composition/mucin.

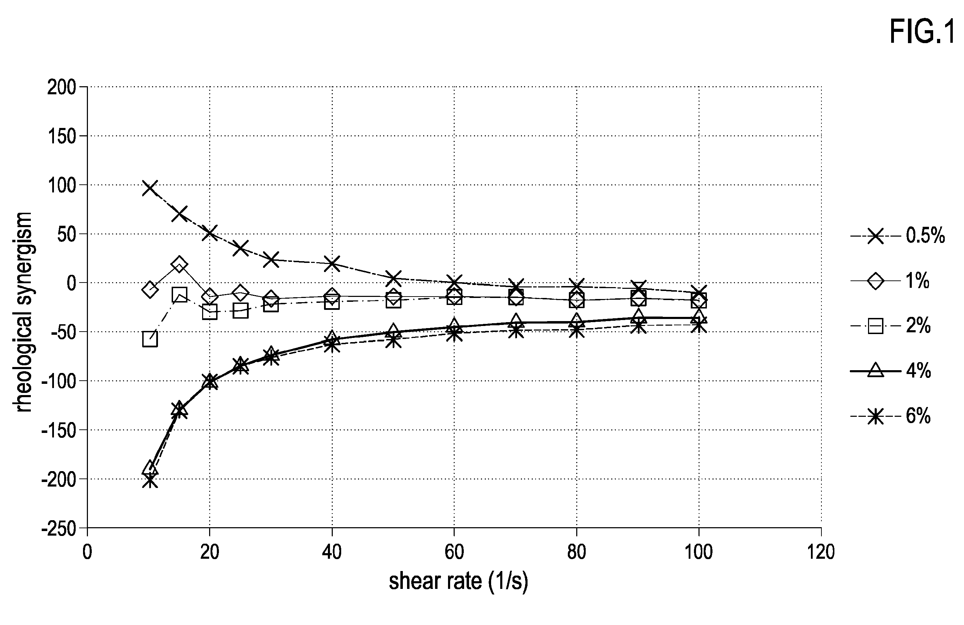

Results are shown in Figure 1, wherein the parameter of rheological synergism (deltaη) of formulations at 6 different mucin concentrations dissolved in water, being measured at different share rates, pH 7 and 37°C is reported.

It is interesting to note that precipitation becomes much more substantial at higher mucin concentrations of 4% and 6% of mucin, being those occurring during common cold, catarrh, nasal infections, allergic rhinitis and sinusitis, when the mucin content in the mucus is greater.

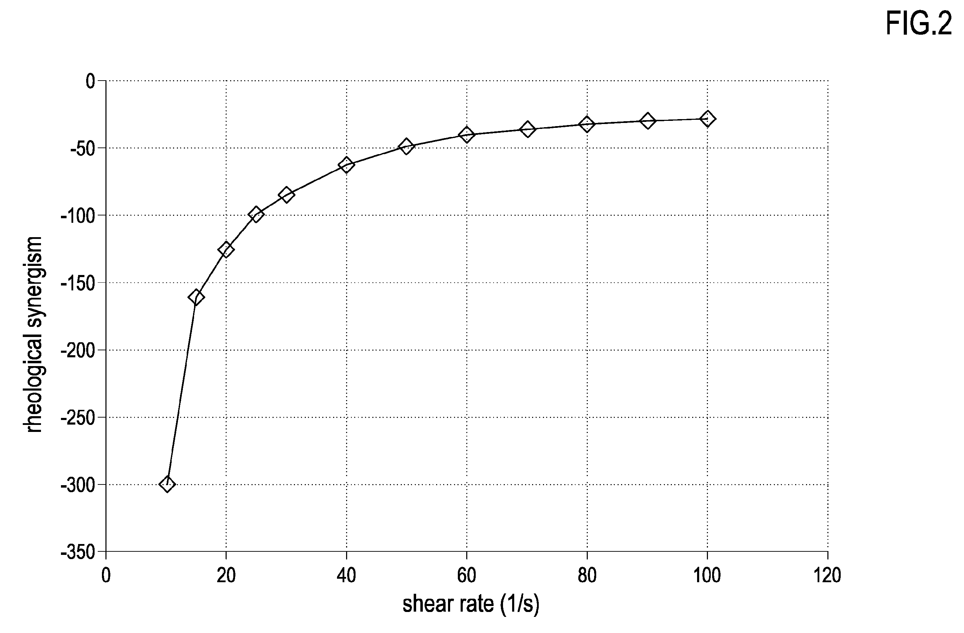

The above measurements were also carried out for a pediatric composition of the present invention containing 50% chitosan and a reduced volume corresponding to 70 microliters, as compared to the previous test, and a "baby-like" mucin. Results are shown in Figure 2, wherein the parameter of rheological synergism (deltaη) of the previous, water-diluted (50%) formulations, mixed with submaxillary gland mucin dissolved in water (1%), measured at different share rates, pH 7 and 37°C is reported.

In both the figures 1 and 2, a negative synergism leading chitosan to sequester mucin is seen.

For example, an adult dose consists of 1 or 2 puffs for a total of 13 microliters of formulation at pH 6-7 which comprises plant chitosan 0.208 mg/dose; one or more cross-linked copolymers of acrylic acid and C10-C30 alkyl acrylates (Acrylates/C10-C30 Alkyl Acrylates Cross polymer) such as for example the emulsifier Pemulen™ TR-2 0.058 mg/dose, 0.045%; water 129.600 mg/dose, 99.645%; preservative 0.180 mg/dose, 0.150%, total 130.000 mg/dose.

For example, a pediatric dose consists of 1 puff for a total of 0.07 microliters of formulation at pH 6-7 which comprises plant chitosan 0.056 mg/dose, 0.080%; one or more cross-linked copolymers of acrylic acid and C10-C30 alkyl acrylates (Acrylates/C10-C30 Alkyl Acrylates Cross polymer) such as for example the emulsifier Pemulen™ TR-2 0.016 mg/dose, 0.022%; water 69.59 mg/dose, 99.75%; preservative 0.180 mg/dose, 0.150%, total 70.000 mg/dose.

An example of composition comprises (by weight):
- plant chitosan: 0.16%,
- emulsifier (i) or (ii): 0.06%,
- water: 99.64%,
- preservative: 0.15%.

## Claims

1. A liquid composition for fluidifying mucus consisting of a mixture comprising or, alternatively, consisting of a plant chitosan and a polymeric emulsifier selected from the group comprising or, alternatively, consisting of cross-linked copolymers of acrylic acid and C10-C30 alkyl acrylates -INCI Name:
Acrylates/C10-30 Alkyl Acrylate Crosspolymer,
for use in the treatment of cold weather ailments, catarrh, common cold, flu, nasal infections, rhinitis, allergic rhinitis and sinusitis.

2. The composition for use according to claim 1, wherein said cross-linked copolymers are selected from the group comprising or, alternatively, consisting of Pemulen™ copolymers or mixtures thereof.

3. The composition for use according to claim 2, wherein said Pemulen™ copolymers is the copolymer Pemulen™ TR-2 Polymeric Emulsifier (INCI Name: Acrylates/C10-30 Alkyl Acrylate Crosspolymer) and/or Pemulen™ TR-2 Polymeric Emulsifier (INCI Name: Acrylates/C10-30 Alkyl Acrylate Crosspolymer).

4. The composition for use according to claims 3 and 4, wherein said Pemulen™ copolymers are in a concentration by weight comprised from 0.01% to 0.5%, preferably from 0.05% to 0.4%; even more preferably from 0.1 % to 0.3%.

5. The composition for use according to claim 1, wherein said composition may further comprise a homopolymer selected from the group comprising or, alternatively, consisting of Carbopol® homopolymers (INCI Name: Carbomer) or mixtures thereof, preferably is Carbopol^{®} Ultrez 30 Polymer.

6. The composition for use according to claim 5, wherein said homopolymer is in a concentration by weight comprised from 0.01% to 1.5%, preferably from 0.05% to 1%; even more preferably from 0.1% to 0.5%.

7. The composition for use according to any one of claims 1-6, wherein said composition is for use as nasal spray, for oral or topical use.

8. The composition for use according to any one of claims 1-7, wherein chitosan is selected from plant chitosans with an average molecular weight comprised from 10 KDa to 80 KDa, preferably from 15 to 50 KDa, even more preferably from 20 KDa to 40 KDa; and having a degree of acetylation comprised from 0 to 50%, preferably from 0 to 30%.

9. A process for preparing a liquid composition according to any one of claims 1-8, wherein said process comprises the following steps:
- in a first vessel a solution comprising water and a polymeric emulsifier selected from cross-linked copolymers of acrylic acid and C10-C30 alkyl acrylates, with a pH comprised from 3 to 5 is prepared;
- in a second vessel a dispersion comprising a plant chitosan and water, with a pH comprised from 8 to 10 is prepared;
- said dispersion of said second vessel is brought to a value comprised from 6.5 to less than 8, by adding a strong inorganic acid, before to adding said dispersion to said solution of said first vessel, to form a stable suspension with a pH value comprised from 6 to 7 and a viscosity value comprised from 50 to 1500 cps.

10. The process according to claim 9, wherein:
- the solution in said first vessel contains water in an amount by weight comprised from 95% to 99.99, preferably from 97 to 99.95%, and contains said polymeric emulsifier in an amount by weight comprised from 0.1 to 0.01%, preferably from 0.08 to 0.02%; said solution in said first vessel is kept under stirring for 1-3 hours at a temperature comprised from 40 to 60°C;
- the solution in said second vessel contains water in an amount by weight comprised from 95 to 99%, preferably from 97 to 99.95%, and contains said plant chitosan in an amount by weight comprised from 0.25 to 0.05%, preferably from 0.08 to 0.02%; said solution in said second vessel is kept under stirring at a temperature comprised from 30 to 40°C;
- said stable suspension being obtained has a starting osmotic concentration value of less than 50 mOsmoles which is then brought to a final osmotic concentration value comprised from 50 mOsmoles and 300 mOsmoles, by adding an inorganic salt, such as NaCl, in an amount by weight comprised from 0.1% to 3%, preferably in an amount by weight comprised from 0.5% to 2.2% by weight, relative to the final weight of the final suspension.
